# EUROPEAN PATENT SPECIFICATION

(11) **EP 1 132 393 B1**
(45) Date of publication and mention of the grant of the patent: **09.04.2003**
(21) Application number: 01108303.7
(22) Date of filing: 15.10.1997
(51) Int. Cl.: C07H 19/056, A61K 31/70

(54) **L-Ribavirin and uses thereof**
L-Ribavirin und dessen Verwendung
L-Ribavirine et son utilisation

(30) Priority: 16.10.1996 US 28585 P
(43) Date of publication of application: 12.09.2001
(62) Divisional of application: 97912762.8
(73) Proprietor: ICN Pharmaceuticals, Inc., Costa Mesa, CA 92626 (US)
(72) Inventor: Ramasamy, Kandasamy, Laguna Hills, CA 92653 (US); Tam, Robert, Irvine, CA 92606 (US); Averett, Devron, Costa Mesa, CA 92626 (US)
(74) Representative: Viering, Jentschura & Partner

(56) References cited:
- WO-A-97/26883
- US-A- 5 559 101
- J CAMPS ET AL: "Ribavirin in the treatment of chronic hepatitis C unresponsive to alpha interferon" JOURNAL OF HEPATOLOGY,XX,XX, vol. 19, no. 3, 1 November 1993 (1993-11-01), pages 408-412, XP002083824
- TAM R C ET AL: "The ribavirin analog ICN 17261 demonstrates reduced toxicity and antiviral effects with retention of both immunomodulatory activity and reduction of hepatitis-induced serum alanine aminotransferase levels" ANTIMICROBIAL AGENTS AND CHEMOTHERAPY,AMERICAN SOCIETY FOR MICROBIOLOGY, WASHINGTON, DC,US, vol. 44, no. 5, 2000, pages 1276-1283, XP002167196 ISSN: 0066-4804

## Description

### FIELD OF THE INVENTION

The present invention relates to the field of L-nucleosides.

### BACKGROUND OF THE INVENTION

The last few decades have seen significant efforts expended in exploring possible uses of D-nucleoside analogs as antiviral agents. Some of this work has borne fruit, and a number of nucleoside analogs are currently being marketed as antiviral drugs, including the HIV reverse transcriptase inhibitors (AZT, ddI, ddC, d4T, and 3TC).

Nucleoside analogs have also been investigated for use as immune system modulators, (Bennet, P. A. et al., *J*. *Med*. *Chem*., 36, 635, **1993**), but again with less than completely satisfactory results. For example, guanosine analogs such as 8-bromo-, 8-mercapto-, 7-methyl-8-oxoguanosine (Goodman, M. G. *Immunopharmacology*, 21, 51-68, **1991**) and 7-thia-8-oxoguanosine (Nagahara, K. *J*. *Med*. *Chem*., 33, 407-415, 1990; U.S, Pat. No. 5,041,426) have been studied over the years for their ability to activate the immune system. These guanosine derivatives show excellent antiviral and/or antitumor activity *in vivo*. But, these C₈-substituted guanosines were unable to activate T-cells (Sharma, B. S. et al., *Clin*. *Exp*. *Metastasis*, 9, 429-439, **1991**). The same was found to be true with 6-arylpyrimidinones (Wierenga, W. *Ann. N*. *Y*. *Acad*. *Sci*., 685, 296-300, **1993**). In other research, a series of 3-deazapurine nucleosides were synthesized and evaluated as immuno-modulating agents. U.S. Patent No. 4,309,419 describes the use of 3-deazaadenosine as being an inhibitor of the immune system. The β-D-nucleoside, β-2'-deoxy-3-deazaguanosine (U.S. Pat. No. 4,950,647) displayed the most potent immunoenhancing potency on activated T-cell response. Antiinflamatory and immunosuppressant activity has also been disclosed for certain 2'-deoxynucleosides (EPO Application 0 038 569). However, these compounds undergo facile in vivo metabolic cleavage of their glycosyl bond, which effectively inactivates their biological potency. Adenosine derivatives disclosed in U.S. Pat. No. 4,148,888 are also catabolized in vivo by deaminase enzymes. In still other research, Levamisole, a thymomimetic immunostimulant (Hadden et al, *Immunol*. *Today*, 14, 275-280, 1993), appears to act on the T-cell lineage in a manner similar to thymic hormones. Tucaresol (Reitz et al, Nature, 377, 71-75,1995), another T-cell stimulant, is now undergoing clinical trials. More recently, 6-substituted purine linker amino acid (Zacharie et al, J. Med. Che., 40, 2883-2894, 1997) has been described as a promising immunostimulant which may be targeted for those disease states which require an increased CTL or Th1 type response.

One possible target of immunomodulation involves stimulation or suppression of Th1 and Th2 lymphokines. Type I (Th1) cells produce interleukin 2 (IL-2), tumor necrosis factor (TNFα) and interferon gamma (IFNγ) and they are responsible primarily for cell-mediated immunity such as delayed type hypersensitivity and antiviral immunity. Type 2 (Th2) cells produce interleukins, IL4, IL-5, IL-6, IL-9, IL-10 and IL-13 and are primarily involved in assisting humoral immune responses such as those seen in response to allergens, e.g. IgE and lgG4 antibody isotype switching (Mosmann, 1989, *Annu Rev Immunol*, 7:145-173). D-guanosine analogs have been shown to elicit various effects on lymphokines IL-1, IL-6, IFNα and TNFα (indirectly) *in vitro* (Goodman, 1988, *Int J Immunopharmacol*, 10, 579-88) and *in vivo* (Smee et al., 1991, *Antiviral Res* 15: ***229**)*. However, the ability of the D-guanosine analogs such as 7- thio-8-oxoguanosine to modulate Type I or Type 2 cytokines directly in T cells was ineffective or has not been described.

Significantly, most of the small molecule research has focused on the synthesis and evaluation of D-nucleosides. This includes Ribavirin (Witkowski, J. T. et al., *J*. *Med*. *Chem.,* 15, 1150, **1972**), AZT (De Clercq, E. *Adv. Drug Res*., 17, 1, 1988), DDI (Yarchoan, R. et al., *Science (Washington*, *D. C.),* 245, 412, **1989**), DDC (Mitsuya, H. et al., *Proc*. *Natl*. *Acad*. *Sci*. *U*. S. *A*., 83, 1911, **1986**), d4T (Mansuri, M. M. et al., *J. Med*. *Chem*., 32, 461, 1989) and 3TC (Doong, S. L. et al., *Proc*. *Natl*. *Acad*. *Sci. U*.*S*.*A*., 88, 8495-8599, **1991**). In this handful of therapeutic agents, only 3TC which contains an unnatural modified L-ribose moiety, the enantiomer of natural D-ribose.

After the approval of 3TC by the FDA, a number of nucleosides with the unnatural L-configuration were reported as having potent chemotherapeutic agents against immunodeficiency virus (HIV), hepatitis B virus (HBV), and certain forms of cancer. These include (-)-□-L-1-[2-(hydroxymethyl)-1,3-oxathiolan-4-yl]-5-fluorocytosine (FTC; Furman, P. A., et al, *Antimicrob*. *Agents Chemother*., 36, 2686-2692, 1992), (-)-□-L-2',3'-dideoxypentofuranosyl-5-flurocytosine (L-FddC; Gosselin, G., et al, *Antimicrob*. *Agents* *Chemother*., 38, 1292-1297, **1994**), (-)-□-L-1-[2-(hydroxymethyl)-1,3-oxathiolan-4-yl]cytosine [(-)-OddC; Grove, **K. L.**, et al, *Cancer Res*., 55, 3008-3011, **1995**], 2',3'-dideoxy-□-L-cystidine (□-L-ddC; Lin, T.S., et al, *J*. *Med*. *Chem.,* 37, 798-803, **1994**), 2'fluoro-5-methyl-□-L-arabinofuranosyluracil (L-FMAU; U.S. Pat. No. 5,567,688), 2',3'-dideoxy-2',3'-didehydro-□-L-cystidine (□-L-d4C; Lin, T.S., et al, *J. Med*. *Chem*., 39, 1757-1759, **1996**), 2',3'-dideoxy-2',3'-didehydro-□-L-5-fluorocystidine (□-L-Fd4C; Lin, T.S., et al, *J. Med. Chem*., 39, 1757-1759, **1996**), L-cyclopentyl carbocyclic nucleosides (Wang, P., et al, *Tetrahedron Letts.,* 38, 4207-4210, **1997**) and variety of 9-(2'-deoxy-2'-fluoro-□-L-arabinofuranosyl)purine nucleosides (Ma, T.' et al, *J. Med. Chem*., 40, 2750-2754, **1997**).

Other research on L-nucleosides has also been reported. U.S. Pat. No. 5,009,698, for example, describes the synthesis and use of L-adenosine to stimulate the growth of a plant. WO 92/08727 describes certain L-2'-deoxyuridines and their use for treating viruses. Spadari, S., et al, *J*. *Med*. *Chem*., 35, 4214-4220, **1992**, describes the synthesis of certain L-β-nucleosides useful for treating viral infections including Herpes Simplex Virus Type I. U.S. Pat. No. 5,559,101 describes the synthesis of α- and β-L-ribofuranosyl nucleosides, processes for their preparation, pharmaceutical composition containing them, and method of using them to treat various diseases in mammals. A German patent (De 195 18 216) describes the synthesis of 2'-fluoro-2'-deoxy-L-β-arabinofuranosyl pyrimidine nucleosides. U.S. Pat. Nos. 5,565,438 and 5,567,688 describe the synthesis and utility of L-FMAU. WO Patent 95/20595 describes the synthesis of 2'-deoxy-2'-fluoro-L-β-arbinofuranosyl purine and pyrimidine nucleosides and method of treating HBV or EBV. U.S. Pat. No. 5,567,689 describes methods for increasing uridine levels with L-nucleosides. WO patent 96/28170 describes a method of reducing the toxicity of D-nucleosides by co-administering an effective amount of L-nucleoside compounds.

Significantly, while some of the known L-nucleosides have shown potent antiviral activity with lower toxicity profiles than their D-counterparts, none of these L-nucleoside compounds have been shown to posses immunomodulatory properties. Moreover, at present there is no effective treatment for the modulation of the immune system where lymphokine profiles (Th1 and Th2 subsets) have been implicated. Thus, there remains a need for novel L-nucleoside analogs, especially a need for L-nucleoside analogs which modulate the immune system, and most especially L-nucleoside analogs which specifically modulate Th1 and Th2

### BRIEF DESCRIPTION OF THE INVENTION

The present invention is directed to 1-β-L-ribofuranosyl-1,2,4-triazole-3-carboxamide (L-Ribavirin), having the following structure:

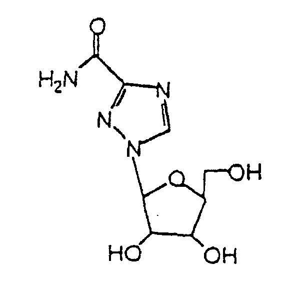

In another aspect of the invention, a pharmaceutical composition comprises a therapeutically effective amount of L-Ribavirin, or a pharmaceutically acceptable ester or salt thereof admixed with at least one pharmaceutically acceptable carrier.

In yet another aspect of the invention, L-Ribavirin is used in the treatment of any condition which responds positively to administration of the compound, and according to any formulation protocol which achieves the positive response. Among other things it is contemplated that L-Ribavirin may be used to treat an infection, an infestation, a cancer or tumor or an autoimmune disease.

### BRIEF DESCRIPTION OF THE FIGURES

Figure 1 is a schematic representation of synthetic chemical steps which may be used to prepare L-Ribavirin as in the examples section below.

Figures 2-3 are graphical representations of the effect of D-Ribavirin and L-Ribavirin on IL-2 TNFα, IFN-γ, IL-4 and IL-5 levels of activated T-cells.

Figure 4 is a graphical representation depicting another set of experiments the effects of L-Ribavirin on the inflammatory ear response to dinitrofluorobenzene were determined.

### DETAILED DESCRIPTION

Where the following terms are used in this specification, they are used as defined below.

The term "nucleoside" refers to a compound composed of any pentose or modified pentose moiety attached to a specific position of a heterocycle or to the natural position of a purine (9-position) or pyrimidine (1-position) or to the equivalent position in an analog.

The term "nucleotide" refers to a phosphate ester substituted on the 5'-position of a nucleoside.

The term "heterocycle" refers to a monovalent saturated or unsaturated carbocyclic radical having at least one hetero atom, such as N, O or S, within the ring each available position of which can be optionally substituted, independently, with, e.g., hydroxy, oxo, amino, imino, lower alkyl, bromo, chloro and/or cyano. Included within this class of substituents are purines, pyrimidines.

The term "purine" refers to nitrogenous bicyclic heterocycles.

The term "pyrimidine" refers to nitrogenous monocyclic heterocycles.

The term "D-nucleosides" that is used in the present invention describes to the nucleoside compounds that have a D-ribose sugar moiety (e.g., Adenosine).

The term "L-nucleosides" that is used in the present invention describes to the nucleoside compounds that have an L-ribose sugar moiety.

The term "L-configuration" is used throughout the present invention to describe the chemical configuration of the ribofuranosyl moiety of the compounds that is linked to the nucleobases. The L-configuration of the sugar moiety of compounds of the present invention contrasts with the D-configuration of ribose sugar moieties of the naturally occurring nucleosides such as cytidine, adenosine, thymidine, guanosine and uridine.

The term "C-nucleosides" is used throughout the specification to describe the linkage type that formed between the ribose sugar moiety and the heterocyclic base. In C-nucleosides, the linkage originates from the C-1 position of the ribose sugar moiety and joins the carbon of the heterocyclic base. The linkage that forms in C-nucleosides are carbon to carbon type.

The term "N-nucleosides" is used throughout the specification to describe the linkage type that formed between the ribose sugar moiety and the heterocyclic base. In N-nucleosides, the linkage originates from the C-1 position of the ribose sugar moiety and joins the nitrogen of the heterocyclic base. The linkage that forms in N-nucleosides are carbon to nitrogen type.

The term "protecting group" refers to a chemical group that is added to, oxygen or nitrogen atom to prevent its further reaction during the course of derivatization of other moieties in the molecule in which the oxygen or nitrogen is located. A wide variety of oxygen and nitrogen protecting groups are known to those skilled in the art of organic synthesis.

The term "lower alkyl" refers to methyl, ethyl, n-propyl, isopropyl, n-butyl, t-butyl, i-butyl or n-hexyl. This term is further exemplified to a cyclic, branched or straight chain from one to six carbon atoms.

The term "aryl" refers to a monovalent unsaturated aromatic carbocyclic radical having a single ring (e.g., phenyl) or two condensed rings (e.g., naphthyl), which can optionally be substituted with hydroxyl, lower alky, chloro, and/or cyano.

The term "heterocycle" refers to a monovalent saturated or unsaturated carbocyclic radical having at least one hetero atom, such as N, O, S, Se or P, within the ring, each available position of which can be optionally substituted or unsubstituted, independently, with e.g., hydroxy, oxo, amino, imino, lower alkyl, bromo, chloro, and/or cyano.

The term "monocyclic" refers to a monovalent saturated carbocyclic radical having at least one hetero atom, such as O, N, S, Se or P, within the ring, each available position of which can be optionally substituted, independently, with a sugar moiety or any other groups like bromo, chloro and/or cyano, so that the monocyclic ring system eventually aromatized [e.g., Thymidine; 1-(2'-deoxy- -D-erythro-pentofuranosyl)thymine].

The term "immunomodulators" refers to natural or synthetic products capable of modifying the normal or aberrant immune system through stimulation or suppression.

The term "effective amount" refers to the amount of L-Ribavirin which will restore immune function to normal levels, or increase immune function above normal levels in order to eliminate infection.

L-Ribavirin has multiple asymmetric centers. Accordingly, it may be prepared in either optically active form or as a racemic mixture. The scope of the invention as described and claimed encompasses the individual optical isomers and non-racemic mixtures thereof as well as the racemic forms of L-Ribavirin.

The terms "α" and "β" indicate the specific stereochemical configuration of a substituent at an asymmetric carbon atom in a chemical structure as drawn. L-Ribavirin described herein is in the L-furanosyl configuration.

The term "enantiomers" refers to a pair of stereoisomers that are non-superimposable mirror images of each other. A mixture of a pair of enantiomers, in a 1:1 ratio, is a "racemic" mixture.

The term "isomers" refers to different compounds that have the same formula. "Stereoisomers" are isomers that differ only in the way the atoms are arranged in space.

A "pharmaceutically acceptable salts" may be any salts derived from inorganic and organic acids or bases.

Ribavirin (1-β-D-ribafuranosyl-1,2,4-triazole-3-carboxamide) is a monocyclic synthetic D-nucleoside that has been demonstrated activity against variety of viral diseases (Huffman et al, *Antimicrob*. *Agents Chemother.,* 3, 235, **1975;** Sidwell et al, *Science,* 177, 705, **1972**) and currently undergoing clinical trials in combination with γ-interferon for the treatment of Hepatitis C virus. In the past two decades, a variety of Ribavirin D-nucleoside analogs have been explored and many of them exhibit the exceptional antiviral and antitumor activities. However, no work has been reported on the synthesis of L-isomer of Ribavirin analogs and their biological activity. In single crystal X-ray analysis Ribavirin resemble structurally to guanosine (Prusiner et al., *Nature*, 244, 166, **1973**). Because of the resemblance of Ribavirin to guanosine, we expected that Ribavirin nucleoside analogs should show similar or superior immuno-modulating activity than guanosine analogs (Robins et al, US 5,041,426) in addition to the antiviral activity.

### Uses

It is contemplated that the L-Ribavirin of the present invention will used to treat a wide variety of conditions, and in fact any condition which responds positively to administration of L-Ribavirin. Among other things it is specifically contemplated that the L-Ribavirin of the invention may be used to treat an infection, an infestation, a cancer or tumor or an autoimmune disease.

Infections contemplated to be treated with the L-Ribavirin of the present invention include respiratory syncytial virus (RSV), hepatitis B virus (HBV), hepatitis C virus (HCV), herpes simplex type I and 2, herpes genitalis, herpes keratitis, herpes encephalitis, herpes zoster, human immunodeficiency virus (HIV), influenza A virus, hantann virus (hemorrhagic fever), human papilloma virus (HPV), measles and fungus.

Infestations contemplated to be treated with the L-Ribavirin of the present invention include protozoan infestations, as well as helminth and other parasitic infestations.

Cancers or tumors contemplated to be treated include those caused by a virus, and the effect may involve inhibiting the transformation of virus-infected cells to a neoplastic state, inhibiting the spread of viruses from transformed cells to other normal cells and/or arresting the growth of virus-transformed cells.

Autoimmune and other diseases contemplated to be treated include arthritis, psoriasis, bowel disease, juvenile diabetes, lupus, multiple sclerosis, gout and gouty arthritis), rheumatoid arthritis, rejection of transplantation, allergy and asthma.

Still other contemplated uses of the L-Ribavirin according to the present invention include use as intermediates in the chemical synthesis of other nucleoside or nucleotide analogs which are, in turn, useful as therapeutic agents or for other purposes.

In yet another aspect, a method of treating a mammal comprises administering a therapeutically and/or prophylactically effective amount of a pharmaceutical containing the L-Ribavirin of the present invention. In this aspect the effect may relate to modulation of some portion of the mammal's immune system, especially modulation of lymphokines profiles of Th1 and Th2. Where modulation of Th1 and Th2 lymphokines occurs, it is contemplated that the modulation may include stimulation of both Th1 and Th2, suppression of both Th1 and Th2, stimulation of either Th1 or Th2 and suppression of the other, or a bimodal modulation in which one effect on Th1/Th2 levels (such as generalized suppression) occurs at a low concentration, while another effect (such as stimulation of either Th1 or Th2 and suppression of the other) occurs at a higher concentration.

In general, the most preferred uses according to the present invention are those in which the L-Ribavirin is relatively less cytotoxic to the non-target host cells and relatively more active against the target. In this respect, it may also be advantageous that L-nucleosides may have increased stability over D-nucleosides, which could lead to better pharmacokinetics. This result may attain because L-nucleosides may not be recognized by enzymes, and therefore may have longer half-lives.

It is contemplated that the L-Ribavirin according to the present invention will be administered in any appropriate pharmaceutical formulation, and under any appropriate protocol. Thus, administration may take place orally, parenterally (including subcutaneous injections, intravenous, intramuscularly, by intrasternal injection or infusion techniques), by inhalation spray, or rectally, topically and so forth, and in dosage unit formulations containing conventional non-toxic pharmaceutically acceptable carriers, adjuvants and vehicles.

By way of example, it is contemplated that L-Ribavirin according to the present invention can be formulated in admixture with a pharmaceutically acceptable carrier. For example, the L-Ribavirin of the present invention can be administered orally as pharmacologically acceptable salts. Because the L-Ribavirin of the present invention is water soluble, it can be administered intravenously in physiological saline solution (e.g., buffered to a pH of about 7.2 to 7.5). Conventional buffers such as phosphates, bicarbonates or citrates can be used for this purpose. Of course, one of ordinary skill in the art may modify the formulations within the teachings of the specification to provide numerous formulations for a particular route of administration without rendering the compositions of the present invention unstable or compromising their therapeutic activity. In particular, the modification of L-Ribavirin to render it more soluble in water or other vehicle, for example, may be easily accomplished by minor modifications (salt formulation, esterification, *etc.)* which are well within the ordinary skill in the art. It is also well within the ordinary skill of the art to modify the route of administration and dosage regimen of L-Ribavirin in order to manage its pharmacokinetics for maximum beneficial effect in patients.

In certain pharmaceutical dosage forms, the pro-drug form of L-Ribavirin, especially including acylated (acetylated or other) derivatives, pyridine esters and various salt forms of L-Ribavirin are preferred. One of ordinary skill in the art will recognize how to readily modify L-Ribavirin to pro-drug forms to facilitate delivery of active compounds to a target site within the host organism or patient. One of ordinary skill in the art will also take advantage of favorable pharmacokinetic parameters of the pro-drug forms, where applicable, in delivering the L-Ribavirin to a targeted site within the host organism or patient to maximize its intended effect.

In addition, L-Ribavirin according to the present invention may be administered alone or in combination with other agents for the treatment of the above infections or conditions. Combination therapies according to the present invention comprise, the administration of L-Ribavirin, or a functional derivative thereof, and at least one other pharmaceutically active ingredient. The active ingredient(s) and pharmaceutically active agents may be administered separately or together and when administered separately this may occur simultaneously of separately in any order. The amounts of the active ingredient(s) and pharmaceutically active agent(s) and the relative timings of administration will be selected in order to achieve the desired combined therapeutic effect. Preferably the combination therapy involves the administration of the L-Ribavirin of the present invention or a physiologically functional derivative thereof and one of the agents mentioned herein below.

Examples of such further therapeutic agents include agents that are effective for the modulation of immune system or associated conditions such as AZT, 3TC, 8-substituted guanosine analogs, 2',3'-dideoxynucleosides, interleukin II, interferons such as Y-interferon, tucaresol, levamisole, isoprinosine and cyclolignans. The L-Ribavirin according to the present invention may be effective for enhancing the biological activity of certain agents according to the present invention by reducing the metabolism or inactivation of other compounds and as such, are co-administered for this intended effect.

With respect to dosage, one of ordinary skill in the art will recognize that a therapeutically effective amount will vary with the infection or condition to be treated, its severity, the treatment regimen to be employed, the pharmacokinetics of the agent used, as well as the patient (animal or human) treated. Effective dosages may range from 1 mg/kg of body weight, or less, to 25 mg/kg of body weight or more. In general a therapeutically effective amount of the present compound in dosage form usually ranges from slightly less than about I mg./kg. to about 25 mg./kg. of the patient, depending upon the condition or infection treated and the route of administration. This dosage range generally produces effective blood level concentrations of active compound ranging from about 0.04 to about 100 micrograms/cc of blood in the patient. It is contemplated, however, that an appropriate regimen will be developed by administering a small amount, and then increasing the amount until either the side effects become unduly adverse, or the intended effect is achieved.

Administration of the active compound may range from continuous (intravenous drip) to several oral administrations per day (for example, Q.I.D.) and may include oral, topical, parenteral, intramuscular, intravenous, sub-cutaneous, transdermal (which may include a penetration enhancement agent), buccal and suppository administration, among other routes of administration.

To prepare the pharmaceutical compositions according to the present invention, a therapeutically effective amount of the L-Ribavirin according to the present invention is preferably intimately admixed with a pharmaceutically acceptable carrier according to conventional pharmaceutical compounding techniques to produce a dose. A carrier may take a wide variety of forms depending on the form of preparation desired for administration, e.g., oral or parenteral. In preparing pharmaceutical compositions in oral dosage form, any of the usual pharmaceutical media may be used. Thus, for liquid oral preparations such as suspensions, elixirs and solutions, suitable carriers and additives including water, glycols, oils, alcohols, flavouring agents, preservatives, colouring agents and the like may be used. For solid oral preparations such as powders, tablets, capsules, and for solid preparations such as suppositories, suitable carriers and additives including starches, sugar carrier, such as dextrose, mannitol, lactose and related carriers, diluents, granulating agents, lubricants, binders, disintegrating agents and the like may be used. If desired, the tablets or capsules may be enteric-coated or sustained release by standard techniques.

For parenteral formulations, the carrier will usually comprise sterile water or aqueous sodium chloride solution, though other ingredients including those which aid dispersion may be included. Of course, where sterile water is to be used and maintained as sterile, the compositions and carriers must also be sterilized. Injectable suspensions may also be prepared, in which case appropriate liquid carriers, suspending agents and the like may be employed.

### Test Results

*In vitro and in vivo* tests on L-Ribavirin, were performed, and the results are described below.

In a first series of experiments, peripheral blood mononuclear cells (PBMCs) were isolated from the buffy coat following Ficoll-Hypaque density gradient centrifugation of 60 ml blood from healthy donors. T-cells were then purified from the PBMCs using Lymphokwik lymphocyte isolation reagent specific for T-cells (LK-25T, One Lambda, Canoga Park CA). An average yield of 40 - 60 x 10⁶ T-cells were then incubated overnight at 37 °C in 20 - 30 ml RPMI-AP5 (RPMI-1640 medium (ICN, Costa Mesa, CA) containing 20 mM HEPES buffer, pH 7.4, 5 % autologous plasma, 1 % L-glutamine, 1 % penicillin/streptomycin and 0.05 % 2-mercaptoethanol) to remove any contaminating adherent cells. In all experiments, T-cells were washed with RPMI-AP5 and then plated on 96-well microtitre plates at a cell concentration of I x 10⁶ cells/ml.

The T-cells were activated by the addition of 500 ng ionomycin and 10 ng phorbol 12-myristate 13-acetate (PMA) (Calbiochem, La Jolla, CA) and incubated for 48 - 72h at 37 °C. PMA/ionomycin-activated T-cells were treated with 0.5 - 50 µM of either Ribavirin (D-Ribavirin) or L-Ribavirin, or with 250 - 10000 U/ml of a control antiviral, interferon-alpha (Accurate, Westbury, NY) immediately following activation and re-treated 24 h later. T-cells from each plate were used for immunofluorescence analysis and the supernatants used for extracellular cytokine measurements. Following activation, 900 µl cell supernatant from each microplate was transferred to another microplate for analysis of cell-derived cytokine production. The cells are then used in immunofluorescence analyses for intracellular cytokine levels and cytokine receptor expression.

Cell-derived human cytokine concentrations were determined in cell supernatants from each microplate. Activation-induced changes in interleukin-2 (IL-2) levels were determined using a commercially available ELISA kit (R & D systems Quantikine kit, Minneapolis, MN) or by.bioassay using the IL-2-dependent cell line, CTLL-2 (ATCC, Rockville, MD). Activation -induced changes in interleukin-4 (IL-4), tumor necrosis factor (TNFα) interleukin-8 (IL-8) (R & D systems (Quantikine kit, Minneapolis, MN) and interferon-gamma (IFN-γ) (Endogen (Cambridge, MA) levels were determined using ELISA kits. All ELISA results were expressed as pg/ml and the CTLL-2 bioassay as counts per minute representing the IL-2-dependent cellular incorporation of ³H-thymidine (ICN, Costa Mesa, CA) by CTLL-2 cells.

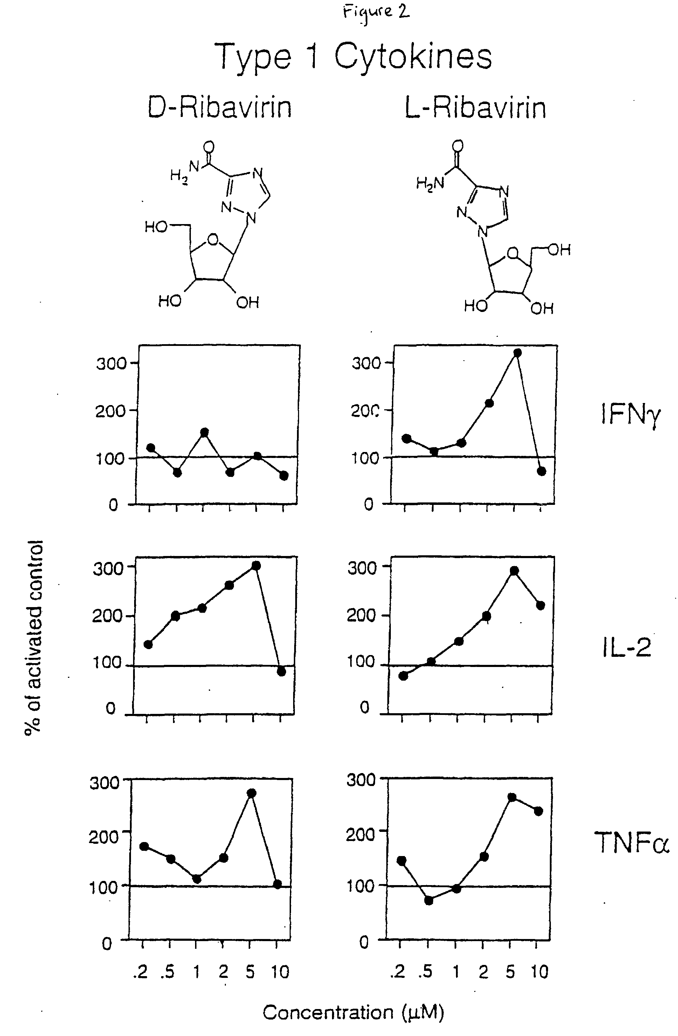

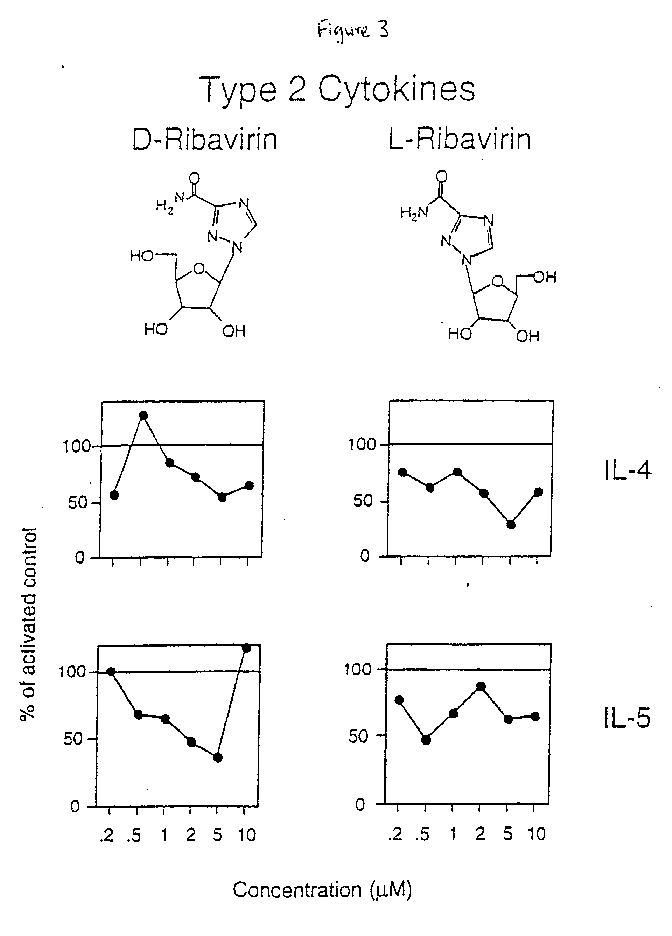

Comparison of the effects of D-Ribavirin and L-Ribavirin (expressed as a percentage of activated control) on IL-2 TNFα, IFN-γ, IL-4 and IL-5 levels are presented in Figures 2 and 3.

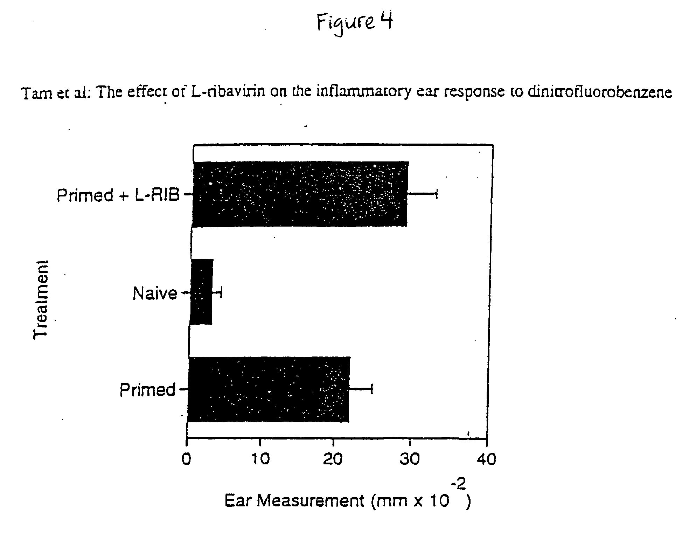

In another set of experiments the effects of L-Ribavirin on the inflammatory ear response to dinitrofluorobenzene were determined. The results of those experiments are shown in Figure 4.

### Synthesis

The L-Ribavirin according to the present invention may be produced according to synthetic methods which are individually readily known to those of ordinary skill in the art. In general, L-Ribavirin according to the present invention are synthesized by condensing appropriate nucleoside base with the necessary sugar synthon to give the protected L-nucleoside which on further manipulation and deprotection of the sugar hydroxyl protecting groups will ultimately give rise to nucleoside analog having the desired ribofuranosyl moiety of the L-configuration.

During chemical synthesis of the L-Ribavirin according to the present invention, one of ordinary skill in the art will be able to practice the present invention, without undue experimentation. In particular, one of ordinary skill in the art will recognize the various steps that should be performed to introduce a particular substituent at the desired position of the base or a substituent at the desired position on the sugar moiety. In addition, chemical steps which are taken to protect functional groups such as hydroxyl or amino groups, among others, as well as de-protected these same functional groups, will be recognized as appropriate within the circumstances of the syntheses.

The invention is further defined by reference to the following examples, which are intended to be illustrative and not limiting. It will be understood by one of ordinary skill in the art that these examples are in no way limiting and that variations of detail can be made without departing from the spirit and scope of the present invention.

The L-Ribavirin of the present invention may be prepared in accordance with well known procedures in the art. Particularly useful is the following synthetic scheme.

Scheme 1: Synthesis of L-Ribavirin precursors: Triazole L-ribofuranosyl nucleosides were prepared by the acid catalyzed fusion procedure (Sato, T., et al, Nippon Kagaku Zasshi, 81, 1440, 1960). Accordingly, the triazoles (**1**) were mixed with 1,2,3,5-tetra-O-acetyl-L-ribose (**2**) and a catalytic amount of bis(p-nitrophenyl)phosphate and heated at 160-165 C for 30 min under reduced pressure to provide the required nucleosides which on further deprotection furnished the L-Ribavirin precursor (**3**). 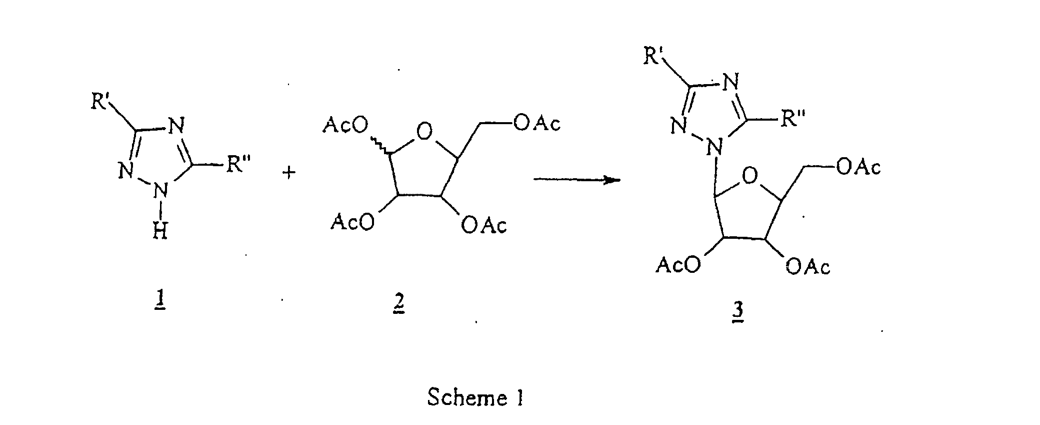 The required 1,2,3,5-tetra-O-acetyl-L-ribose was prepared as shown in Example 2. The hetero monocyclic bases are commercially available from Aldrich, Fluka, ICN, Acros, Alfa, Lancaster and TCI America or were prepared by following the reported procedure that are available in the literature articles (Robins, R.K., et al, Nucleosides & Nucleotides, 13, 17-76, 1994).

Furthermore, substituted sugars such as 1-bromo-2-deoxy-2-fluoro-3,6-O-benzoyl-L-arabinofuranose (Ma, T., et al, *J*. *Med*. *Chem*., 39, 2835-2843, **1996**) and other modified sugars of L-configuration are known in U.S. Pat. No. 5,473,063; WO 96/13512; WO 96/13498; WO 96/22778; WO 95/20595; U.S. 5,473,063; U.S. 5,567,688; WalczaK, K., et al, *Monatsh. fur Chemie,* 123, 349-354(1992); Wengel, J., et al, *J. Org*. *Chem*., 56, 3591-3594 (1991); Genu-Dellac, C., et al, *Tetrahedron Letts.,* 32, 79-82 (1991) and Czernecki, S., et al, *Synthesis,* 783 (1991). In addition, preparation of modified sugars and nucleosides of D-configuration are described in U.S. Pat. No. 5,192,749; WO 94/22890; Uteza, V., et al, *Tetrahedron*, 49, 8579-8588 (1993); Thrane, H., et al, *Tetrahedron*, 51, 10389-10403 (1995); Yoshimura, Y., et al, *Nucleosides* & *Nucleotides,* 14,427-429 (1993; Lawrence, A. J., et al, *J. Org*. *Chem.,* 61, 9213-9222 (1996); Ichikawa, S., et al, *J. Org. Chem*., 62, 1368-1375 (1997); EP 0 457 326 Al; U.S. Pat. No. 3,910,885; WO 96/13498 and Karpeisky, M,Y., et al, *Nucleic Acids Res*. *Symposium Series,* 9, 157 (**1981**). By applying the synthetic procedures (schemes) that has been described in these articles for the preparation of D-nucleosides, the corresponding modified L-nucleosides could also be achieved.

The L-Ribavirin of the invention can be synthesized using the teachings of the schematics provided herein, as well as the specific examples and other schemes set forth below. In addition to the teachings provided herein, the skilled artisan will readily understand how to make the L-Ribavirin of the present invention by applying well known techniques such as those described in Nucleic Acid Chemistry, Improved and New Synthetic Procedures, Methods and Techniques, Edited by Leroy B. Townsend and R. Stuart Tipson, John Wiley & Sons, New York (1978-1991); Chemistry of Nucleosides and Nucleotides, Edited by Leroy B. Townsend, New York, Plenum Press (1988-1994) and Nucleosides and Nucleotides as Antitumor and Antiviral Agents, Edited by Chung K. Chu and David C. Baker, New York, Plenum Press (1993). Suitable methods for making substitution within the sugar moiety of the presently claimed L-Ribavirin are known to those skilled in the art and are described in various publications including: U.S. Pat. No. 5,559,101; U.S. Pat. No. 5,192,749; U.S. Pat. No. 5,473,063; U.S. Pat. No. 5,565,438. Suitable methods for making various heterocyclic compounds and substitution on them are provided in Chemistry of Nucleosides and Nucleotides, Edited by Leroy B. Townsend, New York, Plenum Press, 2, 161-398 (1991) and Chemistry of Nucleosides and in Nucleotides, Edited by Leroy B. Townsend, New York, Plenum Press, 3, 1-535 (1994).

### EXAMPLES

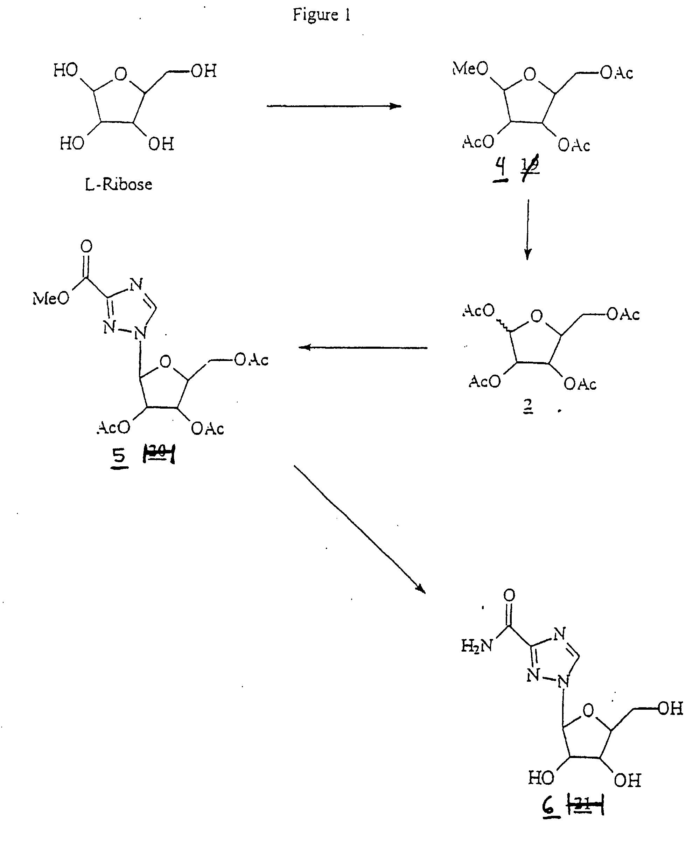

The invention can be further understood by referring to the following examples below, wherein the compound numerals in bold correspond to like numbered numerals in Figure 1.

### EXAMPLE 1

### 1-O-Methyl-2,3,5-Tri-O-acetyl-β-L-ribofuranose(4)

L-Ribose (15.0 g, 100 mmol) was dissolved in dry methanol (200 mL) and cooled to 0 °C. To this cold stirred solution H₂SO₄ (2 mL) was added slowly and the reaction mixture stirred at below 20 °C for 12 h under argon atmosphere. Dry pyridine (75 mL) was added and evaporated to dryness. Dry pyridine (100 mL) was added and evaporated under reduced pressure to an oily residue. This residue was dissolved in dry pyridine (150 mL) and treated with acetic anhydride (50 mL) at 0 °C under argon atmosphere, TEA (41 mL) was added, the reaction stirred at 0 °C for 1 h and at room temperature for 36 h, evaporated to dryness. The residue was dissolved in water (200 mL), solid NaHCO₃ was added slowly to adjust the pH of the solution to 7. The aqueous mixture was extracted in CH₂Cl₂ (250 mL), washed with water (150 mL) and brine (100 mL), dried and concentrated. The oily residue was filtered on a bed of silica gel (200 g), washed with CH₂Cl₂:EtOAc (8:2, 1000 mL). The filtrate was evaporated and the oil was used as such for the next reaction.

### EXAMPLE 2

### 1,2,3,5-Tetra-O-acetyl-β-L-ribofuranose (2)

The syrup (4) (29.0 g, 100 mmol) from the above reaction was co-evaporated with dry toluene (2x100 mL) and dried overnight under solid NaOH at room temperature *in vacuo*. The dried syrup was dissolved in glacial acetic acid (150 mL) and cooled to 0 °C under argon atmosphere. To this cold solution was added acetic anthydride (35 mL) followed by H₂SO₄ (10 mL) very slowly during 15 minute period. The reaction mixture was stirred at room temperature overnight and poured into ice (200 g) with stirring. The mixture was extracted with CHCl₃ (2x200 mL) and the organic extract was washed with water (200 mL), sat. NaHCO₃ (200 mL) and brine (150 mL), dried over anhydrous Na₂SO₄ and evaporated to dryness. The syrup 30 g (94%) that obtained was found to be pure enough for glycosylation reactions.

### EXAMPLE 3

### Methyl 1-(2,3,5-Tri-O-acetyl-β-L-ribofuranosyl)-1,2,4-triazole-3-carboxylate (5)

A mixture of methyl 1,2,4-triazole-3-carboxylate (0.64 g, 5 mmol), 1,2,3,5-tetra-O-acetyl- -L-ribofuranose (2) (1.5 g, 4.72 mmol) and bis(p-nitrophenyl)-phosphate (20 mg) were placed in a pear shaped flask and placed in a preheated oil bath at (160-165 °C). The flask was connected to a water aspirator and kept at 160-165 °C (oil bath temperature) under reduced pressure with stirring for 25 min. The reaction mixture was removed, cooled and diluted with EtOAc ( 150 mL) and sat. NaHCO₃ (100 mL). The product was extracted in EtOAc. The organic extract was washed with water (100 mL) and brine (50 mL), dried and evaporated to dryness. The residue that obtained was purified by flash column of silica gel using CHCl₃→EtOAc as the eluent. The pure fractions were collected and evaporated to dryness to give 1.2 g (66%) of pure product: ¹H NMR (CDCl₃) δ 2.10 (3s, 9H, 3 COCH3), 3.98 (s, 3H, OCH₃), 4.22 (m, 1H), 4.46 (m, 2H), 5.54 (t, 1H), 5.76 (m, 1H), 6.04 (d, 1H, C_{1'}H), and 8.38 (s, 1H, C₃H). Anal. Calc. for C₁₅H₁₉N₃O₉ (385.22): C, 46.75; H, 4.97; N, 10.91. Found: C, 46.82; H, 4.57; N = 10.71.

### EXAMPLE 4

### 1-β-L-Ribofuranosyl-1,2,4-triazole-3-carboxamide (6)

The substrate (5) (1.1 g) was dissolved in CH₃OH/NH₃ at 0 °C and place in a steel bomb. The bomb was closed and stirred at room temperature for 18 h. The steel bomb was cooled, opened and evaporated to dryness. The residue was tried to crystallization with little ethanol. The product crystallized, but on filtration, the crystals re-absorbed water and became a paste. The crystallization repeated several times. Finally it crystallized from Methanol/Ethanol mixture. The colorless crystals was filtered, washed with methanol and dried in *vacuo*. The filtrate was evaporated again which on standing gave further crystals. Total yield 0.5 g (72%); mp: 177.179 °C; [a]_{D}= +38.33 (c 3 mg/mL H₂O); D form of Ribavirin [a]_{D}= -36.0 (c 3.0 mg/mL H₂O); ¹H NMR (Me₂SO-*d*₆) δ 3.46 (m, 1H, C_{5'}H), 3.60 (m, 1H, C₅,H), 3.92 (q, 1H, C₄,H), 4.12 (q, 1H), 4.34 (q, 1H), 4.88 (t, 1H, C_{5'}OH), 5.20 (d, 1H), 5.58 (d, 1H), 5.80 (d, 1H, C_{1'}H), 7.60 (bs, 1H, NH), and 8.82 (s, 1H, C₃H). Anal.Calc. for C₈H₁₂N₄O₅ (244.20): C, 39.34; H, 4.95; N, 22.94. Found: C, 39.23; H, 4.97; N,22.91.

## Claims

1. The compound of L-Ribavirin (1-β-L-ribofuranosyl-1,2,4-triazole-3-carboxamide), having the structural formula:

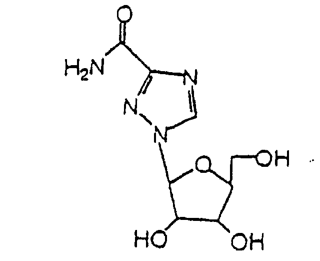

2. The compound of claim 1, wherein the compound comprises an α-nucleoside and β-nucleoside.

3. A pharmaceutical composition comprising the compound of claim 1 or 2, or a pharmaceutically acceptable ester or salt thereof, admixed with at least one pharmaceutically acceptable carrier.

4. Use of a compound of any of claims 1 to 2 for preparing a pharmaceutical composition for treating an inflammatory medical condition which responds positively to administration of said composition.

5. The use of claim 4, wherein the medical condition is selected from the group consisting of an infection, an infestation, a neoplasm and an autoimmune disease.

6. Use of the compound of claim 1 or 2 for the manufacture of a pharmaceutical composition for modulating Th1 and Th2 activities in a patient.

## Patentansprüche

1. Die Verbindung L-Ribavirin (1-β-L-Ribofuranosyl-1,2,4-triazol-3-carboxamid), welche die Strukturformel aufweist:

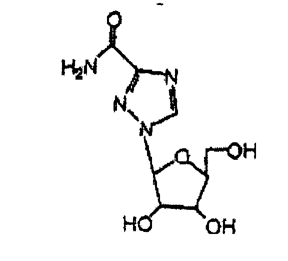

2. Die Verbindung gemäß Anspruch 1, wobei die Verbindung ein α-Nukleosid und β-Nukleosid umfasst.

3. Pharmazeutische Zusammensetzung, welche die Verbindung gemäß Anspruch 1 oder 2 umfasst oder einen pharmazeutisch annehmbaren Ester oder ein Salz davon, gemischt mit zumindest einem pharmazeutisch annehmbaren Trägerstoff.

4. Verwendung einer Verbindung gemäß einem der Ansprüche 1 bis 2 zur Herstellung einer pharmazeutischen Zusammensetzung zur Behandlung eines inflammatorischen medizinischen Zustands, der positiv auf die Verabreichung der Zusammensetzung anspricht.

5. Verwendung gemäß Anspruch 4, wobei der medizinische Zustand aus der Gruppe bestehend aus Infektion, Infestation, Neoplasma und Autoimmunerkrankung ausgewählt wird.

6. Verwendung der Verbindung gemäß Anspruch 1 oder 2 zur Herstellung einer pharmazeutischen Zusammensetzung zur Modulation der Th1- und Th2-Aktivitäten in einem Patienten.

## Revendications

1. Le dérivé (1-β-L-ribofurasonyl-1,2,4-triazole-3-carboxamide), de la L-ribavirine, répondant à la formule développée suivante:

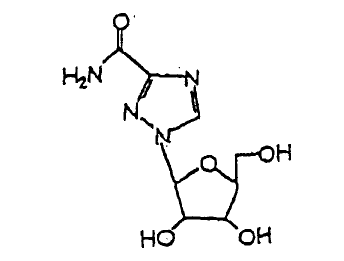

2. Le dérivé selon la revendication 1, dans lequel le dérivé comprend un α-nucléoside et un β-nucléoside.

3. Une composition pharmaceutique comprenant le dérivé selon la renvendication 1 ou 2, ou d'un de ses esters ou sels pharmaceutiquement acceptables, mélangés avec au moins un support pharmaceutiquement acceptable.

4. Utilisation d'un dérivé selon l'une quelconque des revendications 1 ou 2 pour la préparation d'une composition pharmaceutique pour le traitement d'un état médical inflammatoire qui répond positivement à l'administration de ladite composition.

5. L'utilisation selon la revendication 4 dans laquelle l'état médical est choisi dans le groupe consistant en une infection, une infestation, un néoplasme et une maladie autoimmune.

6. L'utilisation du dérivé selon l'une quelconque des revendications 1 ou 2 pour la fabrication d'une composition pharmaceutique pour la modulation des activités Th1 et Th2 chez un patient.
